(19) Europäisches Patentamt
European Patent Office
Office européen des brevets

(11) EP 4 205 766 A1

(12) # EUROPEAN PATENT APPLICATION
published in accordance with Art. 153(4) EPC

(43) Date of publication:
**05.07.2023 Bulletin 2023/27**

(21) Application number: **21861606.8**

(22) Date of filing: **25.08.2021**

(51) International Patent Classification (IPC):
***A61K 45/00*** *(2006.01)* ***A61K 31/404*** *(2006.01)*
***A61P 11/00*** *(2006.01)* ***A61P 43/00*** *(2006.01)*

(52) Cooperative Patent Classification (CPC):
**A61K 31/404; A61K 45/00; A61P 11/00; A61P 43/00**

(86) International application number:
**PCT/JP2021/031131**

(87) International publication number:
**WO 2022/045183 (03.03.2022 Gazette 2022/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.08.2020 JP 2020142467**

(71) Applicants:
- **ONO Pharmaceutical Co., Ltd.**
  **Osaka-shi, Osaka 541-8526 (JP)**
- **Tohoku University**
  **Sendai-shi, Miyagi 980-8577 (JP)**

(72) Inventors:
- **AOKI, Junken**
  **Sendai-shi, Miyagi 980-8577 (JP)**
- **KANO, Kuniyuki**
  **Sendai-shi, Miyagi 980-8577 (JP)**
- **HASHIMOTO, Yasuaki**
  **Mishima-gun, Osaka 618-8585 (JP)**
- **KADODE, Michiaki**
  **Mishima-gun, Osaka 618-8585 (JP)**
- **KAWAHARA, Yuji**
  **Mishima-gun, Osaka 618-8585 (JP)**

(74) Representative: **J A Kemp LLP**
**80 Turnmill Street**
**London EC1M 5QU (GB)**

(54) **PROPHYLACTIC AND/OR THERAPEUTIC AGENT FOR IDIOPATHIC PULMONARY FIBROSIS**

(57) The present invention addresses the problem of providing a prophylactic and/or therapeutic agent for idiopathic pulmonary fibrosis. Provided is a prophylactic and/or therapeutic agent for idiopathic interstitial pneumonia (IPF or the like), comprising a compound having an LPA3 agonistic activity or a salt thereof.

*FIG. 4*

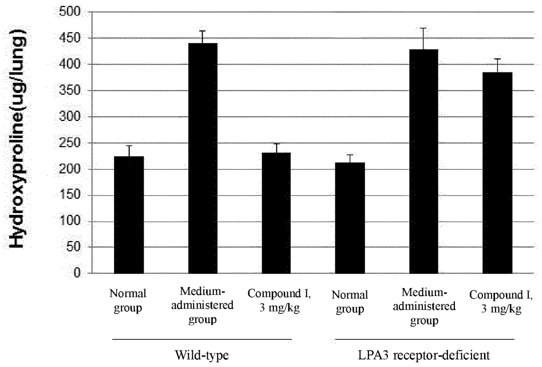


EP 4 205 766 A1

## Description

### TECHNICAL FIELD

This patent application claims priority from prior Japanese Patent Application No. 2020-142467, the entire contents of which are incorporated herein by reference.

In one embodiment, the present invention relates to a prophylactic and/or therapeutic agent for idiopathic pulmonary fibrosis.

### BACKGROUND ART

Idiopathic pulmonary fibrosis (also referred to as "IPF", hereinafter) is the most frequently occurring type of idiopathic interstitial pneumonia and can induce progressive pulmonary fibrosis. The cause for the acute exacerbation of IPF is unknown, and the acute exacerbation of IPF is such a clinical condition that respiratory failure progresses rapidly and the prognosis thereof is poor. The acute exacerbation of IPF is believed to be the predominant cause for the death of an IPF patient. However, the pathological mechanism of this disease has not been elucidated to date.

Meanwhile, lysophosphatidic acid (abbreviated as "LPA", hereinafter) is a phospholipid derivative that can transduce various signals into a cell. It is known that LPA exhibits various pharmacological activities through some G protein-coupled receptors occurring on the surface of a cell. The types of the pharmacological activities induced by LPA include proliferation of cancer cells, invasion of cancer, metastasis of cancer, aggregation of platelets, contraction of smooth muscles, regulation of blood pressure, neuropathic pain and an activity to accelerate would healing. The LPA receptor subtypes are distributed widely in a living body, and the types of the localization of the subtypes are different from each other. Accordingly, it is considered that the roles of the receptors are different among tissues. In the recent studies, it has been found that the LPA receptor has subtypes that have different rolls from each other. The subtypes that are known at the present time are roughly classified into six groups named LPA1, LPA2, LPA3, LPA4, LPA5 and LPA6 (Non-Patent Literature 1, Patent Literature 1). From these facts, it is considered that an antagonist or an agonist for the LPA receptor is useful for the prevention and/or treatment of various diseases associated with the LPA receptor.

In recent years, it has been found that the LPA receptor is involved in a clinical condition of IPF. For example, it has been reported that an LPA1 antagonist (e.g., BMS-986020) is effective as a therapeutic agent for IPF (Non-Patent Literature 2).

It has also been reported that LPA1 and LPA3 antagonists suppress the fibrosis of the lung in a bleomycin-induced scleroderma model (Non-Patent Literature 3).

Furthermore, it has been considered that an inhibitor of autotaxin (ATX) that is involved in the production of LPA is a potential therapeutic agent for IPF (Non-Patent Literature 4).

As the LPA3 agonist, various compounds are known. For example, in paragraphs [0008] to [0036] in Patent Literature 2, compounds respectively having structural formulae (IA) to (IIIA) are disclosed as agonists or antagonist of EDG7 (= LPA3) receptor.

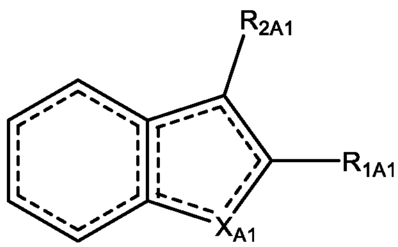

(IA)

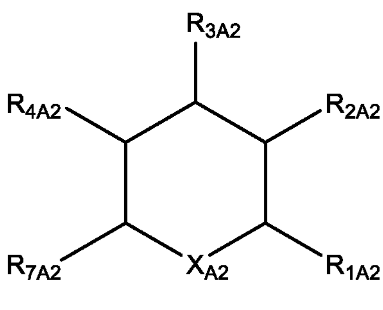

(IIA)

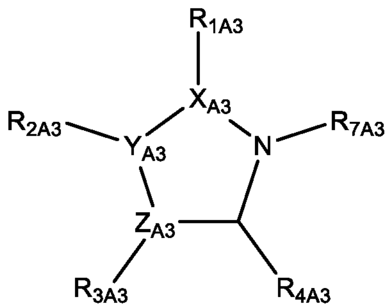

(IIIA)

(wherein $X_{A1}$ represents $NR_{3A1}$, S or O; $R_{1A1}$ represents a hydrogen atom, an alkyl group, a substituted alkyl group or the like; $R_{2A1}$ represents a hydrogen atom, an alkyl group, a substituted alkyl group or the like; $R_{3A1}$ represents a hydrogen atom, an alkyl group, a substituted alkyl group or the like; $R_{1A2}$, $R_{2A2}$, $R_{3A2}$, $R_{4A2}$ and $R_{7A2}$ independently represent -H, -halo, $-NO_2$, -CN or the like; $X_{A2}$ represents $CH_2$, C=O, O or the like; $R_{1A3}$, $R_{2A3}$, $R_{3A3}$, $R_{4A3}$ and $R_{7A3}$ independently represent -H, -halo, $-NO_2$, -CN or the like; and $X_{A3}$, $Y_{A3}$ and $Z_{A3}$ independently represent C=O, O or the like).

In Patent Literature 2, the values of EDG7 (= LPA3) agonistic activities of compounds 127, 129 and 133 are described.

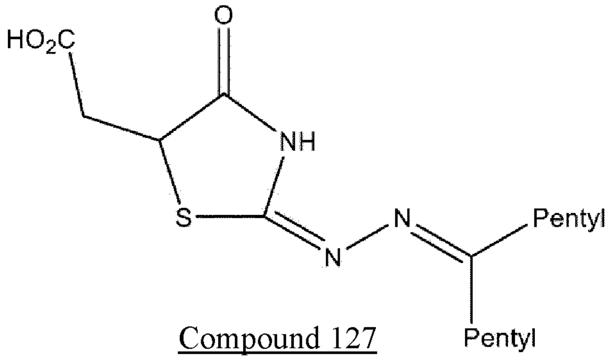

Compound 127

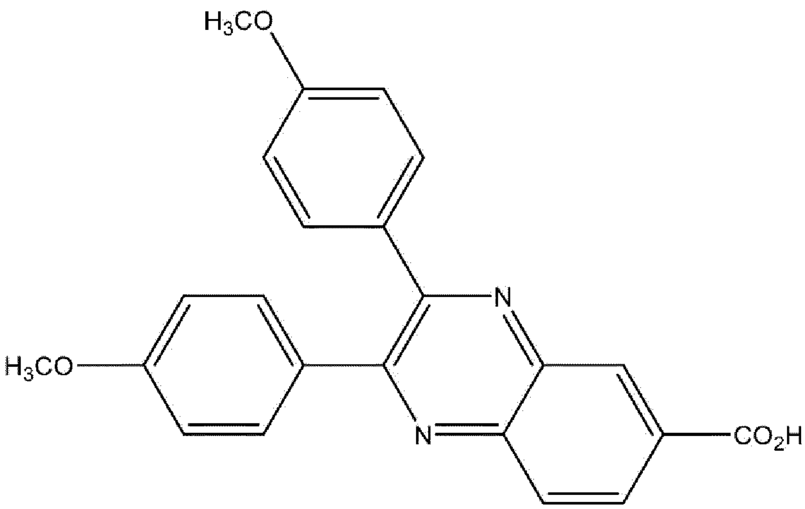

Compound 129

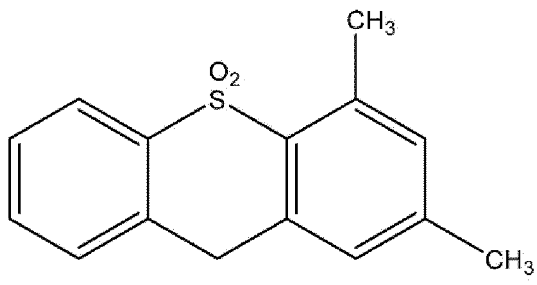

Compound 133

In Non-Patent Literature 5, a thiophosphate derivative shown below, which is named "compound 13", is described as an LPA3 agonist.

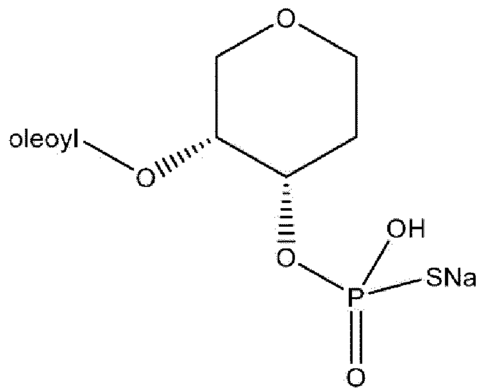

Compound 13

However, the roll of the LPA3 receptor in the clinical condition of IPF has not been found clearly. On the other hand, Patent Literature 2 describes that acute inflammation in acute/chronic lung diseases can be enhanced by an EDG7 (= LPA3) agoni st.

CITATIONS LIST

Patent Literature

Patent Literature 1: Japanese Patent Application Laid-open No. 2008-297278
Patent Literature 2: US Patent Application No. 2005/0261298

Non Patent Literature

Non Patent Literature 1: "Biochemistry", vol. 83, No. 6, p.p. 506-517, 2011
Non Patent Literature 2: CHEST, 2018; 154(5):1061-1069
Non Patent Literature 3: Experimental Dermatology, 2015, 24, 698-702
Non Patent Literature 4: Pulmonary Pharmacology & Therapeutics, Volume 52, October 2018, Pages 32-40
Non Patent Literature 5: Angewandte Chemie, International Edition (2004), 43(21), 2834-2837
Non Patent Literature 6: Consortium of Biological Sciences 2017 (ConBio2017), 3PT12-12 (3P-0035)
Non Patent Literature 7: The 93th Annual Meeting of the Japanese Biochemical Society, program abstracts, Page [P-088] (2020.09)

## SUMMARY OF INVENTION

### TECHNICAL PROBLEMS

The present invention addresses the problem of providing a prophylactic and/or therapeutic agent for IPF, particularly a prophylactic and/or therapeutic agent for suppressing pulmonary fibrosis.

### SOLUTIONS TO PROBLEMS

In order to solve the problem, the present inventors have made intensive and extensive studies on the pathological mechanism of IPF and LPA agonists. As a result, it is found that an LPA3 agonist can be applied to the prevention and/or treatment of diseases associated with pulmonary fibrosis (e.g., idiopathic interstitial pneumonia (IPF)).

The present invention includes the following aspects.

[1] A prophylactic and/or therapeutic agent for a disease associated with pulmonary fibrosis (e.g., idiopathic interstitial pneumonia or pulmonary fibrosis associated with systemic sclerosis), the prophylactic and/or therapeutic agent containing a compound having an LPA3 agonistic activity or a salt thereof.

[2] The prophylactic and/or therapeutic agent according to [1], wherein the disease associated with pulmonary fibrosis is idiopathic interstitial pneumonia.

[3] The prophylactic and/or therapeutic agent according to [2], wherein the idiopathic interstitial pneumonia is idiopathic pulmonary fibrosis.

[4] The prophylactic and/or therapeutic agent according to any one of [1] to [3], wherein the prophylactic and/or therapeutic agent suppresses fibrosis.

[5] The prophylactic and/or therapeutic agent according to any one of [1] to [4], wherein the compound having an LPA3 agonistic activity or a salt thereof is 3-[(cyclobutylamino)methyl]-1-(4-methylbenzyl)-1H-indol-2-carboxylic acid (also abbreviated as "Compound I", hereinafter) or a salt thereof.

[6] A pulmonary fibrosis suppressing agent containing a compound having an LPA3 agonistic activity or a salt thereof.

[7] A pharmaceutical composition for preventing and/or treating a disease associated with pulmonary fibrosis, the pharmaceutical composition containing a compound having an LPA3 agonistic activity or a salt thereof.

[8] A method for preventing and/or treating a disease associated with pulmonary fibrosis, containing administering an effective amount of a compound having an LPA3 agonistic activity or a salt thereof to a mammal.

[9] A compound having an LPA3 agonistic activity or a salt thereof for use for the prevention and/or treatment of a disease associated with pulmonary fibrosis.

[10] A use of a compound having an LPA3 agonistic activity or a salt thereof for the manufacture of a prophylactic and/or therapeutic agent for a disease associated with pulmonary fibrosis.

### ADVANTAGEOUS EFFECTS OF INVENTION

In one embodiment, the present invention can provide a prophylactic and/or therapeutic agent for IPF, particularly a prophylactic and/or therapeutic agent for suppressing fibrosis in IPF.

### BRIEF DESCRIPTION OF DRAWINGS

Fig. 1 shows that Compound I prevents the increase in the weight of the lung in bleomycin-induced pulmonary fibrosis models produced using male ICR mice.

Fig. 2 shows that Compound I prevents the increase in the amount of hydroxyproline in bleomycin-induced pulmonary fibrosis models produced using male ICR mice.

Fig. 3 shows that Compound I does not prevent the increase in the weight of the lung in bleomycin-induced pulmonary fibrosis models produced using LPA3 receptor-deficient C57BL/6N mice.

Fig. 4 shows that Compound I does not prevent the increase in the amount of hydroxyproline in bleomycin-induced pulmonary fibrosis models produced using LPA3 receptor-deficient C57BL/6N mice.

DESCRIPTION OF EMBODIMENTS

[LPA3 agonist]

The term "compound having an LPA3 agonistic activity" (also referred to as "LPA3 agonistic compound", hereinafter) as used herein is not particularly limited, as long as the compound has an LPA3 agonistic activity. For example, the compound includes, within the scope thereof, compounds 127, 129 and 133 disclosed in Patent Literature 2 and compound 13 disclosed in Non-Patent Literature 5. The compound includes the compounds that have been discovered to date, and also includes compounds that will be discovered in the future.
For example, the LPA3 agonistic compound can be screened using an evaluation system described in Biological Example 1 mentioned below. Examples of the LPA3 agonistic compound include compounds each of which shows an $EC_{50}$ value of 1 μmol/L or less in the evaluation system described in Biological Example 1.
In one embodiment, the LPA3 agonistic compound is a compound showing an LPA3-selective agonistic activity. An example of the compound showing an LPA3-selective agonistic activity is a compound having selectivity to LPA1 and/or LPA2. In one embodiment, the compound showing an LPA3-selective agonistic activity is a compound that has selectivity to LPA1 and/or LPA2 by 10 times or more higher, 20 times or more higher, 50 times or more or higher, or 100 times or more higher, than the selectivity to other receptors. In one embodiment, the selectivity can be determined by comparing the $EC_{50}$ values for the individual receptors with each other.
One example of the compound is the following compound. 3-[(Cyclobutylamino)methyl]-1-(4-methylbenzyl)-1H-indol-2-carboxylic acid

(Compound I)

Compound I can be produced in accordance with the below-mentioned method. Compound I is a compound showing an LPA3-selective agonistic activity. In the below-mentioned LPA agonistic activity measurement experiment (Biological Example 1), Compound I showed an $EC_{50}$ value of 0.06 μmol/L for LPA3, while no agonistic activity of Compound I was not observed for LPA1 and LPA2 (in which the agonistic activity for each of LPA1 and LPA2 was > 10 μmol/L).
The LPA3 agonistic compound in the present invention includes, within the scope thereof, isomers thereof, unless otherwise specified. For example, an alkyl group, an alkylene group, an alkenylene group, an alkynylene group and the like include linear types and branched types thereof. Furthermore, all of double bond localization isomers (an E, Z, cis or trans form), isomers each based on an asymmetric carbon atom (an R- or S form, an α- or β-form, an enantiomer, a diastereomer), optically active compounds each having optical rotation (a D-, L-, d- or l-form), polar compounds produced by chromatographic separation (a highly polar compound, a lowly polar compound), equilibrium compounds, rotational isomers, and mixtures each comprising the aforementioned compounds at an arbitrary ratio, and racemic mixtures are included within the scope of the present invention. In the present invention, all of tautomeric isomers are also included.
The LPA3 agonistic compound can be converted to a salt thereof by a known method. The LPA3 agonistic compound can also be produced in the form of a salt, depending on the production method to be employed, the type of a starting substance and the like.
As the salt, a pharmaceutically acceptable salt is preferred.
The salt is preferably water-soluble.
Examples of the salt include an acid addition salt, an alkali metal salt, an alkaline earth metal salt, an ammonium salt, and an amine salt.
Examples of the acid addition salt include: an inorganic acid salt such as a hydrochloric acid salt, a hydrobromic acid salt, a hydroiodic acid salt, a sulfuric acid salt, a phosphoric acid salt and a nitric acid salt; and an organic acid salt such as an acetic acid salt, a lactic acid salt, a tartaric acid salt, a benzoic acid salt, a citric acid salt, a methanesulfonic acid salt, an ethanesulfonic acid salt, a trifluoroacetic acid salt, a benzenesulfonic acid salt, a toluenesulfonic acid salt, an isethionic acid salt, a glucuronic acid salt and a gluconic acid salt.
Examples of the alkali metal salt include a potassium salt and a sodium salt.
Examples of the alkaline earth metal include a calcium salt and a magnesium salt.
An example of the ammonium salt is a tetramethylammonium salt.
Examples of the amine salt include a triethylamine salt, a methylamine salt, a dimethylamine salt, a cyclopentylamine salt, a benzylamine salt, a phenethylamine salt, a piperidine salt, a monoethanolamine salt, a diethanolamine salt, a tris(hydroxymethyl)aminomethane salt, a lysine salt, an arginine salt and an N-methyl-D-glucamine salt.
Each of the LPA3 agonistic compound and the salt thereof may be a hydrate, a non-hydrate, a solvate or a non-solvate. The solvate is preferably non-toxic and soluble in water. A proper example of the solvate is an alcohol-based solvent(e.g., ethanol).

The LPA3 agonistic compound may be in the form of a prodrug.

The term "prodrug of the LPA3 agonistic compound" refers to a compound that is converted to the LPA3 agonistic compound through a reaction with an enzyme or gastric acid *in vivo.* Examples of the prodrug of the LPA3 agonistic compound include: when the LPA3 agonistic compound has an amino group, a compound having such a structure that the amino group in the LPA3 agonistic compound is acylated, alkylated or phosphorylated (e.g., a compound having such a structure that the amino group in the LPA3 agonistic compound is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, or tert-butylated); when the LPA3 agonistic compound has a hydroxyl group, a compound having such a structure that the hydroxyl group in the LPA3 agonistic compound is acylated, alkylated, phosphorylated or borated (e.g., a compound having such a structure that the hydroxyl group in the LPA3 agonistic compound is acetylated, palmitoylated, propanoylated, pivaloylated, succinylated, fumarylated, alanylated or dimethylaminomethylcarbonylated); and when the LPA3 agonistic compound has a carboxyl group, a compound having such a structure that the carboxyl group in the LPA3 agonistic compound is esterified or amidated (e.g., a compound having such a structure that the carboxyl group in the LPA3 agonistic compound is ethylesterified, phenylesterified, carboxymethylesterified, dimethylaminomethylesterified, pivaloyloxymethylesterified, 1-{(ethoxycarbonyl)oxy}ethylesterified, phthalidylesterified, (5-methyl-2-oxo-1,3-dioxylen-4-yl)methylesterified, 1-{[(cyclohexyl oxy)carbonyl]oxy}ethylesterified or methylamidated). These compounds can be produced by known methods. The prodrug of the LPA3 agonistic compound may be either one of a hydrate or a non-hydrate. The prodrug of the LPA3 agonistic compound may be a compound that can be converted to the LPA3 agonistic compound under physiological conditions as described in "Drug Development", vol. 7, article "Molecular Design", pp. 163-198, published by Hirokawa Shoten, 1990.

Each of atoms constituting the LPA3 agonistic compound may be substituted by an isotope thereof (e.g., $^{2}H$, $^{3}H$, $^{13}C$, $^{14}C$, $^{15}N$, $^{16}N$, $^{17}O$, $^{18}O$, $^{18}F$, $^{35}S$, $^{36}Cl$, $^{77}Br$, $^{125}I$).

Among the LPA3 agonistic compounds, a compound having an optical activity can be produced using a starting material or reagent having an optical activity, or can be produced by the optical resolution of an intermediate for the production of a racemic compound thereof, or can be produced by the optical resolution of a racemic compound thereof.

The methods for the optical resolution are known. For example, a method can be mentioned, in which a complex or salt of the intermediate for the production of the racemic compound or the racemic compound with another optically active compound is formed and then the desired compound is isolated by recrystallization or is separated directly using a chiral column or the like.

In each of the reactions mentioned in the present description, a reaction accompanied by heating may be performed using a water bath, an oil bath, a sand bath or microwaves, as known by the persons skilled in the art.

In each of the reactions mentioned in the present description, it is possible to appropriately use a solid-phase-supported reagent that is supported on a high-molecular-weight polymer (e.g., polystyrene, polyacrylamide, polypropylene, polyethylene glycol).

In each of the reactions mentioned in the specification, a reaction product can be purified by a conventional purification means, such as distillation under ambient pressure or under reduced pressure, high-performance liquid chromatography using silica gel or magnesium silicate, thin-layer chromatography, an ion exchange resin, a scavenger resin, column chromatography, washing and recrystallization. The purification may be carried out in every reactions, or may be carried out after the completion of several reactions.

[Toxicity]

The toxicity of the LPA3 agonistic compound or a pharmaceutically acceptable salt thereof is low. Therefore, the LPA3 agonistic compound or a pharmaceutically acceptable salt thereof can be used as a drug safely.

[Medical use]

The present invention relates to a prophylactic and/or therapeutic agent for idiopathic interstitial pneumonia (preferably IPF), the prophylactic and/or therapeutic agent comprising the LPA3 agonistic compound or a salt thereof (wherein both of the compounds are collectively referred to as "LPA3 agonist", hereinafter) and is effective for the prevention and/or treatment of idiopathic interstitial pneumonia (preferably IPF). As the tissue change occurring in IPF, fibrosis, invasion of inflammatory cells, and growth of fibroblasts are known, for example. As the clinical conditions of IPF, cough, dyspnea, loss of body weight, malaise, fatigue, and acute exacerbation with worsened enhanced dyspnea are known, for example. The prophylactic and/or therapeutic agent according to the present invention is effective for the suppression of these tissue changes and the prevention and/or treatment of these symptoms, and is particularly effective for the suppression of fibrosis.

The present invention can suppress fibrosis in the lung. Accordingly, the present invention also relates to a pulmonary fibrosis suppressing agent and/or a prophylactic and/or therapeutic agent for a disease associated with

fibrosis, which contains the LPA3 agonist as an active ingredient and is therefore effective for the prevention and/or treatment of a disease associated with pulmonary fibrosis.

In the present invention, examples of the disease associated with pulmonary fibrosis include idiopathic interstitial pneumonia (preferably IPF) and pulmonary fibrosis associated with systemic sclerosis. One embodiment of the disease associated with pulmonary fibrosis is idiopathic interstitial pneumonia (preferably IPF).

The term "prevention" as used herein refers to the suppression of the onset of a disease in a subject which is not affected by the disease yet.

The term "treatment" as used herein refers to the amelioration of a symptom of a disease, the prevention of the increase in severity of the disease, the retention of remission of the disease, the prevention of exacerbation of the disease, or the prevention of recurrence of the disease in a subject which has been affected by the disease.

The wording "'suppression' of fibrosis" includes the decrease in the amount of fibrosis compared with the case where the LPA3 agonist is not used.

The terms "subject" and "patient" as used herein include a mammal (e.g., human, monkey, dog, feline, rabbit, rat, mouse). Preferably, the subject or patient is a human.

In the present invention, the LPA3 agonist may comprise a single substance or a combination of two or more substances.

In the present invention, the dosage amount of the LPA3 agonist may vary depending on the age, the body weight, the physical condition, the therapeutic effect, the route of administration, the time period of treatment and the like of the subject or patient. In general, the LPA3 agonist is administered orally at a dosage amount of 0.1 ng to 1000 mg in a single daily dose or several divided doses for an adult person, or is administered parenterally at a dosage amount of 0.1 ng to 10 mg in a single daily dose or several divided doses for an adult person, or is administered continuously intravenously for 1 to 24 hours per day. As mentioned above, the dosage amount varies depending on various factors. Therefore, a smaller dosage amount may be enough in some cases, or a dosage amount larger than the above-mentioned dosage amounts may be necessary in some cases.

In the present invention, the LPA3 agonist may be administered as a combined preparation in combination with another drug, for the purpose of:

1) the supplementation and/or enhancement of the prophylactic and/or therapeutic effect of the compound;
2) the improvement of the pharmacokinetics/absorption of the compound or the reduction in the amount of the compound to be administered; and/or
3) the reduction in adverse side effects of the compound.

In the present invention, the combined preparation of the LPA3 agonist and another drug may be administered in the form of a formulated preparation in which both of the components are blended in a single preparation. Alternatively, preparations of the components may be administered separately. In the case where the drugs are administered as separately prepared preparations, the types administration includes simultaneous administration and sequential administration. In the case of sequential administration, the LPA3 agonist may be administered first, and another drug may be administered later; or another drug may be administered first and the LPA3 agonist may be administered later. In this case, the methods for the administration of the components may be the same as or different from each other.

In the present invention, the mass ratio between the LPA3 agonist and another drug is not particularly limited.

In the present invention, another drug to be administered may comprise a combination of two or more components.

In the present invention, another drug to be used for the supplementation and/or enhancement of the prophylactic and/or therapeutic effect of the LPA3 agonist against a disease associated with pulmonary fibrosis includes drugs that have been discovered to date as well as drugs that will be discovered in the future on the basis of the above-mentioned mechanism.

When the LPA3 agonist is used alone as a single-component drug or is used as a combined preparation in combination with another drug for the purpose of the prevention and/or treatment of the above-mentioned disease, it is generally performed to formulate the LPA3 agonist that serves as an active ingredient together with a pharmaceutically acceptable carrier such as an additive and a solvent and administer the formulated preparation systemically or topically through an oral or parenteral route. The term "pharmaceutically acceptable carrier" as used herein refers to a substance which is commonly used in the formulation of a drug and is different from an active ingredient. The pharmaceutically acceptable carrier is preferably a substance which does not exert any pharmacological activity in the amount contained in the preparation to be administered, is nontoxic, and cannot interfere with the therapeutic effect of the active ingredient. The pharmaceutically acceptable carrier can also be used for the purpose of increasing the usefulness of the active ingredient and the preparation, making the production of the preparation easy, stabilizing the quality of the preparation, improving the usability of the preparation, or the like. More specifically, a substance as described in, for example, "Japanese Pharmaceutical Excipients Directory" (edited by Japan Pharmaceutical Excipients Council), published in

2000 by Yakuji Nippo Limited may be selected appropriately depending on the intended use.

Examples of the dosage form to be employed for the administration include a preparation for oral administration (e.g., tables, capsules, granules, a powder, an oral liquid, a syrup, an oral jelly), a preparation for administration to oral cavity (e.g., tables for oral cavity administration, a spray for oral cavity administration, a semi-solid preparation for oral cavity administration, a gargle), an injection preparation (e.g., an injection), a preparation for dialysis (e.g., a medicine for dialysis), a preparation for inhalation (e.g., an inhalant), an ophthalmic preparation (e.g., eye drops, an ophthalmic ointment), an otologic preparation (e.g., ear drops), a rhinologic preparation (e.g., nose drops), a rectal preparation (e.g., a suppository, a rectal semi-solid preparation, an enema), a vaginal preparation (e.g., vaginal tables, a vaginal suppository), and a preparation for skin (e.g., a solid preparation for external application, a liquid for external application, a spray, an ointment, a cream, a gel, an adhesive skin patch).

As another embodiment, the present invention also includes a pharmaceutical composition for preventing and/or treating a disease associated with pulmonary fibrosis, which contains the LPA3 agonist. The pharmaceutical composition may also contain a pharmaceutically acceptable carrier in addition to the LPA3 agonist.

In another embodiment, the present invention includes a use of an LPA3 agonist for the manufacture of a prophylactic and/or therapeutic agent for a disease associated with pulmonary fibrosis.

In still another embodiment, the present invention includes an LPA3 agonist that is intended to be used for the prevention and/or treatment of a disease associated with pulmonary fibrosis.

In further another embodiment, the present invention includes a method for preventing and/or treating a disease associated with pulmonary fibrosis, which comprises administering an effective amount of the LPA3 agonist to a patient who needs the prevention and/or treatment of the disease associated with pulmonary fibrosis. The term "effective amount" as used herein refers to an amount at which a disease associated with pulmonary fibrosis can be prevented and/or treated.

In these embodiments, the requirements for the LPA3 agonist and the like are as described in detail about the above-mentioned prophylactic and/or therapeutic agent for a disease associated with pulmonary fibrosis.

Unless otherwise specified, all of the technical and scientific terms and the abbreviated terms used herein have the same meanings as those which are understood commonly by persons skilled in the art to which the present invention pertains.

In the present description, the entire contents of all of the Patent Literatures, the Non-Patent Literatures and the reference documents which are explicitly cited in the present description can be incorporated herein by reference.

## EXAMPLES

The present invention will be described in details by way of reference to examples. However, the present invention is not limited to these examples.

The compounds mentioned in the present invention and the compounds shown in examples are named by using ACD/Name (ver. 6.00, Advanced Chemistry Development Inc.) or Chemdraw Ultra (ver. 12.0, Cambridge Soft Corp.).

LCMS was performed using Waters i-class (A) or Shimadzu Nexera X2 (B) system under the following conditions. Column: YMC Triart C 18 2.0 mm × 30 mm, 1.9 μm; flow rate: 1.0 mL/min.; temperature: 30°C; mobile phase A: a 0.1% aqueous trifluoroacetic acid (abbreviated as "TFA", hereinafter) solution; mobile phase B: a 0.1% TFA acetonitrile solution: gradient (a (mobile phase (A)) : (mobile phase (B)) ratio was mentioned): (0 to 0.10 minutes): (95% : 5%); 0.10 to 1.20 minutes: (95% : 5%) to (5% : 95%); 1.20 to 1.50 minutes: (5% : 95%).

The numerical values shown with respect to NMR are measurement values (chemical shift values) of $^{1}$H-NMR wherein a measurement solvents shown in the corresponding section was used.

### Reference Example 1: Ethyl 3-formyl-1H-indol-2-carboxylate

A solution of phosphorus oxychloride (2.7 mL) in dimethylformamide (abbreviated as "DMF", hereinafter) (8 mL) was stirred for 30 minutes under an ice bath, then a solution of ethyl indol-2-carboxylate (5.0 g) in DMF (8.5 mL) was added to the solution, and the resultant reaction mixture was stirred at room temperature for 30 minutes and then at 60°C for 4 hours. The reaction mixture was cooled to room temperature, then water (30 mL) was poured into the reaction solution, and then 2N sodium hydroxide was added to the resultant solution to neutralize the solution, thereby producing a solid material. The solid material was filtrated, and the resultant product was washed with DMF/water (2/1) and was then dried to produce the title compound (5.5 g) having the following physical property values.

TLC : Rf 0.23 (hexane : ethyl acetate = 4 : 1);
$^{1}$H-NMR ($CDCl_3$): δ 1.48, 4.53, 7.31-7.50, 8.45-8.53, 9.31, 10.76.

Reference Example 2: Ethyl 3-formyl-1-(4-methylbenzyl)-1H-indol-2-carboxylate

Potassium carbonate (1.9 g) and 1-(bromomethyl)-4-methylbenzene (940 mg) were added to a solution of the compound (1.0 g) produced in Reference Example 1 in DMF (10 mL), and the resultant reaction mixture was stirred at room temperature for 1 hour. Water was added to the reaction mixture, and the resultant solution was extracted with ethyl acetate. An organic layer was dried over sodium sulfate and was then concentrated under a reduced pressure. A residue thus produced was purified by silica gel column chromatography (hexane : ethyl acetate = 19 : 1 → 9 : 1 → 4:1) to produce the title compound (1.41 g) having the following physical property values. TLC : Rf 0.38 (hexane : ethyl acetate = 4 : 1);
$^1$H-NMR ($CDCl_3$): δ 1.39, 2.29, 4.45, 5.78, 6.96, 7.08, 7.32-7.43, 8.50-8.57, 10.64.

Reference Example 3: Ethyl

3-[(cyclobutylamino)methyl]-1-(4-methylbenzyl)-1H-indol-2-carboxylate

Cyclobutylamine (155 mg) and acetic acid (0.5 mL) were added to a solution of the compound (350 mg) produced in Reference Example 2 in 1,2-dichloroethane (5 mL), and the resultant solution was stirred at 45°C for 5 hours. The reaction mixture was cooled to room temperature, then sodium triacetoxyborohydride (462 mg) was added to the reaction mixture, and the resultant solution was stirred at room temperature overnight. Water and a saturated aqueous sodium bicarbonate solution were added to the reaction mixture, and the resultant solution was extracted with ethyl acetate. An organic layer was dried over sodium sulfate and was then concentrated under a reduced pressure. A residue thus produced was purified by silica gel column chromatography (hexane : ethyl acetate = 1 : 1 → 0 : 1) to produce the title compound (330 mg) having the following physical property values.

TLC : Rf 0.59 (chloroform : methanol = 9: 1);
$^1$H-NMR ($CDCl_3$): δ 1.36, 1.52-1.80, 2.07-2.20, 3.30-3.44, 4.14, 4.36, 5.73, 6.89, 7.03, 7.13-7.22, 7.25-7.37, 7.78.

Example 1: 3-[(Cyclobutylamino)methyl]-1-(4-methylbenzyl)-1H-indol-2-carboxylic acid (Compound I)

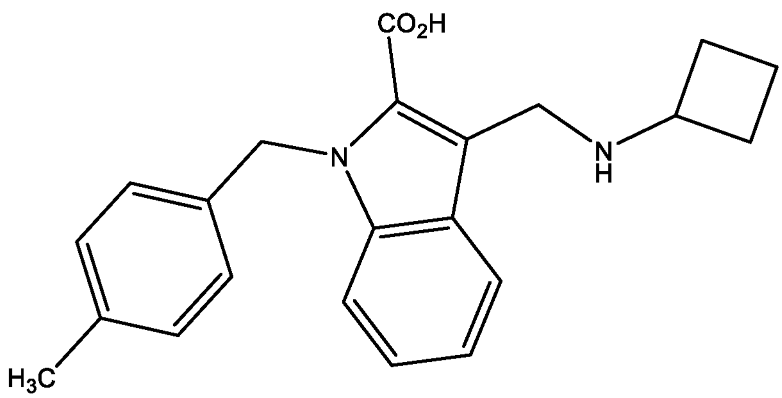

An aqueous 2N sodium hydroxide solution (4.5 mL) was added to a solution of the compound (330 mg) produced in Reference Example 3 in methanol (9 mL) and 1,2-dimethoxyethane (4.5 mL), and the resultant reaction mixture was stirred at 50°C for 3 hours. The reaction mixture was cooled to room temperature and was then neutralized with 2N hydrochloric acid (4.5 mL), then water was added to the resultant solution to produce a solid material, and then the solid material was filtrated and was then dried to produce a title compound (250 mg) having the following physical property values.

LCMS (A) retention time (min.): 0.79;
MS (ESI, Pos.): 349 $(M+H)^+$.
$^1$H-NMR (DMSO-$d_6$): δ 1.70-1.87, 2.13-2.25, 3.60-3.75, 4.32, 5.91, 6.99, 7.05, 7.16, 7.26, 7.51, 7.81, 10.00.

Hereinbelow, biological experimental examples are shown, and the effect of the LPA3 agonist was confirmed on the basis of these experimental methods.

Biological Example 1: LPA3 agonistic activity measurement experiment using lysophosphatidic acid receptor subtype-expressing cell

[Operations]

An RH7777 cell derived from rat hepatocellular carcinoma was used as a host, and a cell strain in which a human LPA3 receptor was expressed stably (Multispan, Inc., Cat No.C1053-6) was used in the experiment. Cells that had been cultured to subconfluence were detached and were then suspended in a culture medium (DMEM containing 10% of FBS, 1% of penicillin/streptomycin, and 30μg/mL of puromycin) in such a manner that the concentration of the cells became $4 \times 10^5$ cells/mL. The culture was seeded in a 96-well optical bottom plate (Asahi Techno Glass Corporation, MT4940-010) in an amount of 100 μL per well, and the cells were cultured for 1 day under the conditions including 5% $CO_2$ and 37°C.

On the day of measurement, the culture medium was removed, then a culture medium containing 0.2% of FBS was added to the wells in an amount of 100 μL per well, and then the plate was subjected to culture for 4 to 6 hours. Subsequently, the culture medium was removed, then an assay buffer (Hank's Balanced Salt Solution containing 0.02 mol/L of HEPES, 2.5 mmol/L of probenecid, 0.1% of BSA, 5 μmol/L of Fluo-4, Quenching Buffer, and Pluronic F-127) was added to the wells in an amount of 100 μL per well, and then the plate was incubated under 5% $CO_2$, at 37°C for about 1 hour. The plate was set in a fluorescence drug screening system (Hamamatsu Photonics K.K.), then a cell addition solution containing only a test substance or dimethyl sulfoxide (abbreviated as "DMSO", hereinafter) was added to the wells in an amount of 25 μL per well 30 seconds after the start of the measurement, then a ligand was added to the wells in an amount of 25 μL per well after 3 minutes, and then the measurement was performed for 4 minutes. A fluorescence wavelength 540 nm was measured at an excitation wavelength of 480 nm, and then the concentration of $Ca^{2+}$ in a cell was determined as a change in fluorescence intensity.

The agonistic activity of a test substance was calculated as a Ratio elevation degree after the addition of the test substance, in which the Ratio elevation value of the cells into which the cell addition solution containing only DMSO, which was shown upon the addition of the ligand, was defined as 100%. An $EC_{50}$ value was calculated as a 50% acting concentration from activities (%) before and after the agonistic activity reached more than 50% using Microsoft Excel.

[Results]

In the LPA3 agonistic activity measurement experiment, the compound produced in Example 1 (Compound I) showed an $EC_{50}$ value of 0.06 μmol/L, and therefore showed a potent LPA3 agonistic activity.

In the measurement method, the LPA1 or LPA2 agonistic activity can be measured by using a cell strain (Multispan, Inc., Cat No.C1048-6, C1050-6) in which a human LPA1 receptor or LPA2 receptor has been expresses stably in place of the cell strain in which the human LPA3 receptor has been expressed stably, or by changing the above-mentioned conditions on the basis of the common knowledge of the persons skilled in the art.

Biological Example 2: Efficacy in mice experimentally bleomycin-induced pulmonary fibrosis models (Examination using male ICR mice)

[Operations]

Male ICR mice (Charles River Laboratories Japan, Inc., the week age at the start of experiment: 12-week old) were used. Bleomycin hydrochloride (Nippon Kayaku Co., Ltd.) was dissolved in physiological saline (Otsuka Pharmaceutical Factory, Inc.) to prepare a 3-mg/mL solution. The bleomycin solution thus prepared was administered into a tail vein of each of the mice at a dosage volume of 5 mL/kg at a frequency of 1 time/day for 5 days. For a normal group, physiological saline was administered into a tail vein at a dosage volume of 5 mL/kg at a frequency of 1 time/day for 5 days.

On the day of first administration of bleomycin, the body weights were measured, and the mice were divided into groups equally in such a manner that a significant difference was not observed in the average values of the body weights among the groups. After the grouping, the administration of a test substance or the medium started, and the administration was performed repeatedly through oral route at a frequency of two times/day from the day of the first administration of bleomycin to day 28. The volume of a solution to be administered was calculated on the basis of the body weight of each of the mice.

On the next day of the day of the final administration, each of the animals was subjected to the opening of the abdominal cavity under the anesthesia with 2% isoflurane, and then the abdomen aorta in each of the mice was dissected to bleed to death. After the bleeding to death, the right lung was excised, and the lung wet weight was measured. Subsequently, the tissues of the right lung were dried with a dry-heat sterilizer, and the dry weight of the right lung was measured. Subsequently, 7.2-N hydrochloric acid (2 mL) was added to the dried right lung, and the resultant product

was hydrolyzed in an autoclave at about 121°C for 30 minutes. Subsequently, the hydrolyzed product was neutralized with 2.5 N sodium hydroxide. The weight of the product after the neutralization was measured to calculate the amount (g) of the sample after the neutralization. The sample after the neutralization was diluted with water for injection appropriately, and a portion (2 mL) of the solution was used as a sample for the measurement of the content of hydroxyproline (HYP).

A chloramine T reagent (1 mL) was added to an HYP standard solution (2 mL), the resultant mixture was left to stand for 20 minutes at room temperature, then a perchloric acid reagent (1 mL) was added to the solution, and then the resultant mixture was left to stand for 5 minutes at room temperature. A p-dimethylaminobenzaldehyde reagent (1 mL) was added to the solution, then the resultant mixture was warmed to 60°C for 20 minutes, and then the absorbance of a supernatant produced by the filtration of the mixture through a filter was measured at 557 nm with a spectrophotometer to produce a standard curve.

With respect to each of the HYP content measurement samples (2 mL), the measurement of the absorbance was performed in the same manner as mentioned above, and then the HYP content (μg/mL) was calculated on the basis of the standard curve.

If necessary, each the HYP content measurement samples was diluted for the measurement. The HYP amount in the right lung (μg/Lung) was calculated in accordance with the following equation.

$$[\text{HYP amount } (\mu g/mL)] \times [\text{dilution factor}] \times [\text{amount (g) of sample after neutralization}] = [\text{amount } (\mu g/Lung) \text{ of HYP in right lung}]$$

[Results]

The weights of the right lung and the HYP amounts in the right lung in the normal group, the control group and the test substance-administered group (Example 1: 0.3 mg/kg, 1 mg/kg, and 3 mg/kg) are shown in Figs. 1 and 2, respectively. The weight of the right lung and the HYP amount in the right lung in the test substance-administered group were lower compared with those in the medium-administered group.

From these results, it was clearly demonstrated that the compound produced in Example 1 (Compound I) that is the LPA3 agonist significantly prevented the increase in the weight of the right lung and the HYP amount in the right lung in the mouse experimentally bleomycin-induced pulmonary fibrosis model.

Biological Example 3: Efficacy in mice experimentally bleomycin-induced pulmonary fibrosis models (Examination using LPA3 receptor-deficient C57BL/6N mice)

[Operations]

Wild-type and LPA3 receptor-deficient male C57BL/6N mice (Oriental BioService, Inc., the week age at the start of the experiment: 13-week old) were used. Bleomycin hydrochloride (Nippon Kayaku Co., Ltd.) was dissolved in physiological saline (Otsuka Pharmaceutical Factory, Inc.) to prepare a 0.41-mg/mL solution. The bleomycin solution thus prepared was administered to each of the mice at a dosage volume of 75 μL through a transpulmonary route. For a normal group, physiological saline was administered at a dosage volume of 75 μL through a transpulmonary route.

On the day of first administration of bleomycin, the body weights were measured, and the mice were divided into groups equally in such a manner that a significant difference was not observed in the average values of the body weights among the groups. After the grouping, the administration of a test substance or the medium started, and the administration was performed repeatedly through an oral route at a frequency of two times/day from the day of the first administration of bleomycin to day 21. The volume of a solution to be administered was calculated on the basis of the body weight of each of the mice.

On the next day of the day of the final administration, each of the animals was subjected to the opening of the abdominal cavity under the anesthesia with a three types of mixed anesthetic agent (the intraperitoneal administration of 0.3 mg/10 mL/kg of medetomidine, 4 mg/10 mL/kg of midazolam, and 5 mg/10 mL/kg of butorphanol), and then the abdomen aorta in each of the animals was dissected to bleed to death. After the bleeding to death, the lung was excised, and the lung wet weight was measured. Subsequently, the lung was frozen. The frozen lung was homogenized, and then 11.2-N hydrochloric acid (200 μL) was added to a homogenate solution to hydrolyze the homogenate solution at about 120°C for 34 hours. The content of hydroxyproline in the solution after the hydrolysis was measured using a hydroxyproline quantification kit (BioVision, Inc.).

[Results]

The lung weights and the HYP amounts in the lung in the wild-type mice normal group, the wild-type mice control group, the wild-type mice test substance-administered group (Example 1: 3 mg/kg), an LPA3 receptor-deficient mice normal group, an LPA3 receptor-deficient mice control group, an LPA3 receptor-deficient mice test substance-administered group (Example 1: 3 mg/kg) are shown in Figs. 3 and 4, respectively. For the wild-type mice, the lung weight and the HYP amount in the lung in the test substance-administered group were smaller compared with those in the medium-administered group. For the LPA3 receptor-deficient mice, the lung weight and the HYP amount in the lung in the test substance-administered group did not show significant difference compared with those in the medium-administered group.

From these results, it was clearly demonstrated that the efficacy of the compound produced in Example 1 (Compound I) that was the LPA3 agonist relied on the LPA3 receptor.

From Biological Example 2 and Biological Example 3, it was clearly demonstrated that the compound having an LPA3 agonistic activity (LPA3 agonist) had an activity to suppress fibrosis in IPF and was therefore useful for the prevention and/or treatment of IPF.

INDUSTRIAL APPLICABILITY

In one embodiment, the present invention provides a prophylactic and/or therapeutic agent for IPF, particularly a prophylactic and/or therapeutic agent for suppressing fibrosis in IPF, and is therefore useful.

## Claims

1. A prophylactic and/or therapeutic agent for idiopathic interstitial pneumonia, comprising a compound having an LPA3 agonistic activity or a salt thereof.

2. The prophylactic and/or therapeutic agent according to claim 1, wherein the idiopathic interstitial pneumonia is idiopathic pulmonary fibrosis.

3. The prophylactic and/or therapeutic agent according to claim 1 or 2, wherein the prophylactic and/or therapeutic agent suppresses fibrosis.

4. The prophylactic and/or therapeutic agent according to any one of claims 1 to 3, wherein the compound having an LPA3 agonistic activity or a salt thereof is 3-[(cyclobutylamino)methyl]-1-(4-methylbenzyl)-1H-indol-2-carboxylic acid or a salt thereof.

5. A pulmonary fibrosis suppressing agent comprising a compound having an LPA3 agonistic activity or a salt thereof.

6. A pharmaceutical composition for preventing and/or treating idiopathic interstitial pneumonia, comprising a compound having an LPA3 agonistic activity or a salt thereof.

7. A method for preventing and/or treating idiopathic interstitial pneumonia, comprising administering an effective amount of a compound having an LPA3 agonistic activity or a salt thereof to a mammal.

8. A compound having an LPA3 agonistic activity or a salt thereof for use for the prevention and/or treatment of idiopathic interstitial pneumonia.

9. A use of a compound having an LPA3 agonistic activity or a salt thereof for the manufacture of a prophylactic and/or therapeutic agent for idiopathic interstitial pneumonia.

FIG. 1

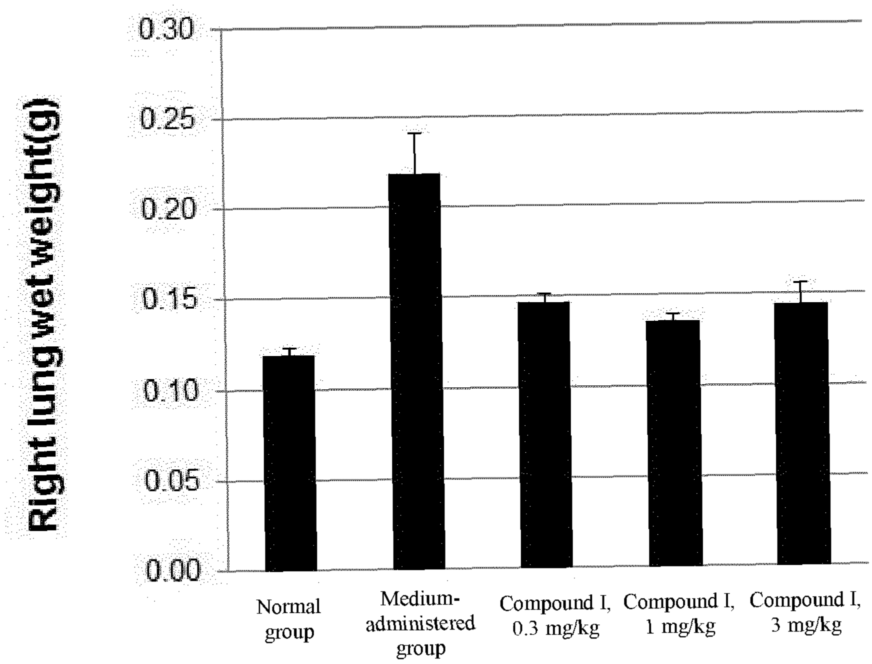
Right lung wet weight(g)
0.30
0.25
0.20
0.15
0.10
0.05
0.00
Normal group
Medium-administered group
Compound I, 0.3 mg/kg
Compound I, 1 mg/kg
Compound I, 3 mg/kg

FIG. 2

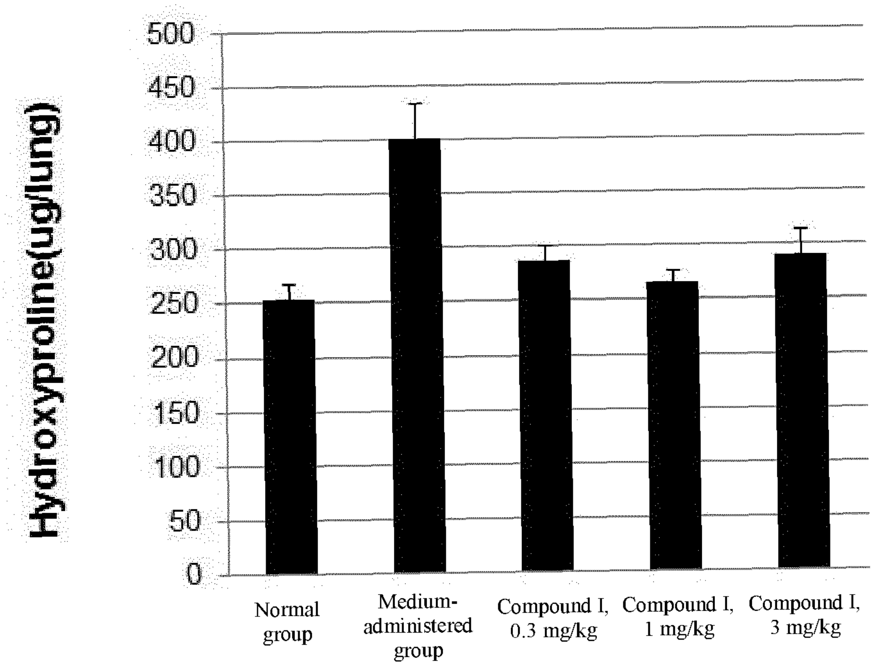
Hydroxyproline(ug/lung)
500
450
400
350
300
250
200
150
100
50
0
Normal group
Medium-administered group
Compound I, 0.3 mg/kg
Compound I, 1 mg/kg
Compound I, 3 mg/kg

FIG. 3

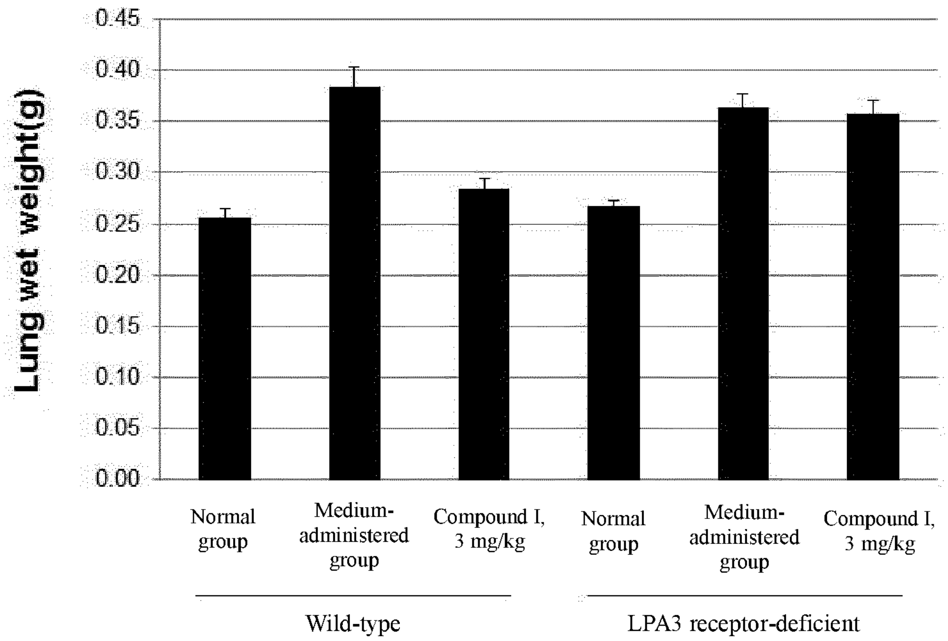
0.45
0.40
0.35
0.30
0.25
0.20
0.15
0.10
0.05
0.00
Lung wet weight(g)
Normal group
Medium-administered group
Compound I, 3 mg/kg
Normal group
Medium-administered group
Compound I, 3 mg/kg
Wild-type
LPA3 receptor-deficient

FIG. 4

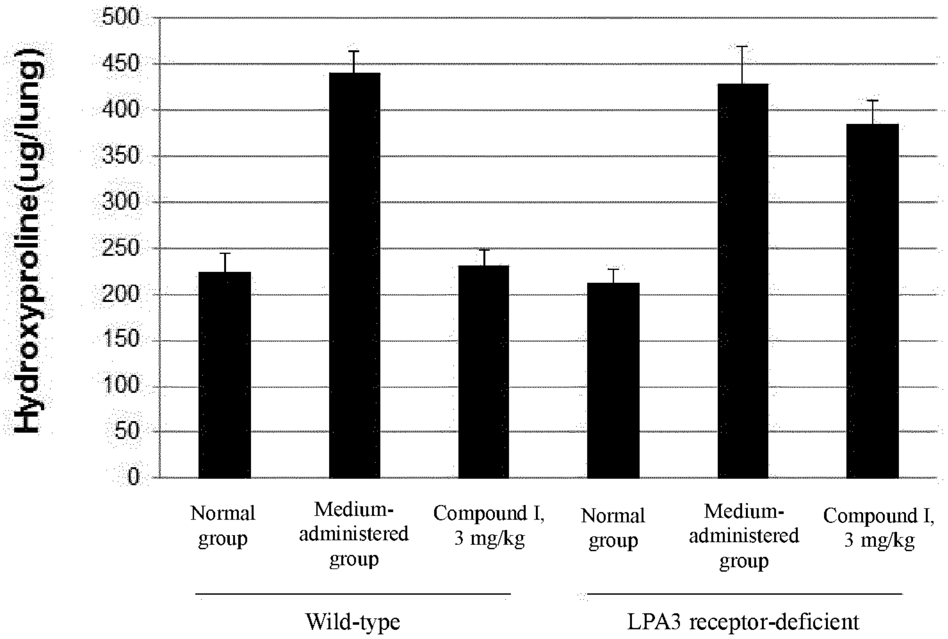
500
450
400
350
300
250
200
150
100
50
0
Hydroxyproline(ug/lung)
Normal group
Medium-administered group
Compound I, 3 mg/kg
Normal group
Medium-administered group
Compound I, 3 mg/kg
Wild-type
LPA3 receptor-deficient

## INTERNATIONAL SEARCH REPORT

International application No.
**PCT/JP2021/031131**

### A. CLASSIFICATION OF SUBJECT MATTER

***A61K 45/00***(2006.01)i; ***A61K 31/404***(2006.01)i; ***A61P 11/00***(2006.01)i; ***A61P 43/00***(2006.01)i
FI: A61K45/00; A61P11/00; A61K31/404; A61P43/00 111

According to International Patent Classification (IPC) or to both national classification and IPC

### B. FIELDS SEARCHED

Minimum documentation searched (classification system followed by classification symbols)

A61K45/00; A61K31/404; A61P11/00; A61P43/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | 冬室 若菜, 他2名, リゾホスファチジン酸受容体LPA3作動薬の肺線維症モデルに対する薬効の解析, 第93回日本生化学会大会プログラム集, 25 August 2020, 2Z10-06(P-088) in particular, methods, results and discussion, (FUYUMURO, Wakana et al. Effects of LPA3 agonist in pulmonary fibrosis model.), non-official translation (Programs of the 93rd Annual Meeting of the Japanese Biochemical Society) | 1-3, 5-9 |
| X | IM, D. Pharmacological tools for lysophospholipid GPCRs: development of agonists and antagonists for LPA and S1P receptors. Acta Pharmacologica Sinica., 2010, 31, pp. 1213-1222 table 1 | 8 |
| A | | 1-7, 9 |
| X | YUNG, Y. C. STODDARD, N. C. CHUN, J. LPA receptor signaling: pharmacology, physiology, and pathophysiology. J. Lipid Res., 2014, 55, pp. 1192-1214 table 2 | 8 |
| A | | 1-7, 9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 October 2021** | **09 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/031131**

**C. DOCUMENTS CONSIDERED TO BE RELEVANT**

| Category* | Citation of document, with indication, where appropriate, of the relevant passages | Relevant to claim No. |
|---|---|---|
| A | 大前 直樹, 他3名, 新規特発性肺線維症急性増悪モデルにおけるLPA3の保護作用, 2017年生命科学系学会合同年次大会(ConBio2017)プログラム, 2017, 3PT12-12(3P-0035) entire text, (OMAE, Naoki et al.), non-official translation (Protective effect of LPA3 in a model for acute exacerbation of novel Idiopathic pulmonary fibrosis. Program of the Consortium of Biological Sciences 2017 (ConBio2017).) | 1-9 |
| A | LEUSCHEN, B. A. SCHULTE, N. A. TOEWS, M. L. Lysophosphatidic acid (LPA) receptor roles in LPA and serum stimulation of lung fibroblast proliferation. The FASEB Journal., 2009, 23(S1), p.753.3 entire text | 1-9 |
| P, X | WO 2021/107125 A1 (ONO PHARMACEUTICAL CO) 03 June 2021 (2021-06-03) example 2, biological example 1 | 8 |

Form PCT/ISA/210 (second sheet) (January 2015)

**INTERNATIONAL SEARCH REPORT**
**Information on patent family members**

International application No.
**PCT/JP2021/031131**

| Patent document cited in search report | Publication date (day/month/year) | Patent family member(s) | Publication date (day/month/year) |
|---|---|---|---|
| WO 2021/107125 A1 | 03 June 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020142467 A
- JP 2008297278 A
- US 20050261298 A


### Non-patent literature cited in the description


- *Biochemistry,* 2011, vol. 83 (6), 506-517
- *CHEST,* 2018, vol. 154 (5), 1061-1069
- *Experimental Dermatology,* 2015, vol. 24, 698-702
- *Pulmonary Pharmacology & Therapeutics,* October 2018, vol. 52, 32-40
- *Angewandte Chemie, International Edition,* 2004, vol. 43 (21), 2834-2837
- *The 93th Annual Meeting of the Japanese Biochemical Society,* September 2020, 088
- Molecular Design. Drug Development. Hirokawa Shoten, 1990, vol. 7, 163-198
- Japanese Pharmaceutical Excipients Directory. Yakuji Nippo Limited, 2000